# FASCICULE DE BREVET EUROPEEN

(11) **EP 0 925 779 B1**
(45) Date de publication et mention de la délivrance du brevet: **04.02.2004**
(21) Numéro de dépôt: 98402958.7
(22) Date de dépôt: 26.11.1998
(51) Int. Cl.: A61K 7/48

(54) **Composition cosmétique contenant de l'acide cinnamique ou d'au moins l'un de ses dérivés et son utilisation**
Zimtsäure oder mindestens ein Zimtsäurederivat enthaltende kosmetische Zusammensetzung und ihre Verwendung
Cosmetic composition containing cinnamic acid or at least a derivative and its use

(30) Priorité: 19.12.1997 FR 9716178
(43) Date de publication de la demande: 30.06.1999
(73) Titulaire: L'OREAL, 75008 Paris (FR)
(72) Inventeur: Breton, Lionel, 78000 Versailles (FR); Girerd-Dugue, Florence, 34130 Maugio (FR); Renault, Béatrice, 94410 Saint Maurice (FR)
(74) Mandataire: Tezier Herman, Béatrice

(56) Documents cités:
- EP-A- 0 664 290
- EP-A- 0 716 847
- US-A- 5 093 109
- DATABASE WPI Week 8802 Derwent Publications Ltd., London, GB; AN 88-012502 XP002081837 "Hair rinsing composition contains hops, nettle and chestnut extracts, safflower concentrate, sunflower oil cake protein production byproduct and isopropyl palmitate" & SU 1 311 734 A (BIOKHIMREAKTIV COMB) 23 mai 1987
- PATENT ABSTRACTS OF JAPAN vol. 017, no. 674 (C-1140), 10 décembre 1993 & JP 05 221845 A (HISAMITSU PHARMACEUT CO INC), 31 août 1993
- PATENT ABSTRACTS OF JAPAN vol. 018, no. 118 (C-1172), 25 février 1994 & JP 05 310526 A (EISAI CO LTD), 22 novembre 1993
- PATENT ABSTRACTS OF JAPAN vol. 017, no. 055 (C-1023), 3 février 1993 & JP 04 266807 A (SOUKEN KK), 22 septembre 1992
- DATABASE WPI Week 8936 Derwent Publications Ltd., London, GB; AN 89-258961 XP002081819 "Cosmetic material giving whitening effect to skin - contains mistures of specified faty acid derivatives, any one or more or ascorbic acid, placenta extract, kojic acid, glucosamine, etc." & JP 01 186811 A (SUNSTAR KK) 26 juillet 1989
- W.A. POUCHER: "Poucher's Perfumes, Cosmetics and Soaps. Volume 1" , CHAPMAN & HALL , LONDON XP002081818 * page 84-89 *
- P. ROVESTI: "Recherches sur l'action des auxines et des phytohormones en cosmétique" PARFUMERIE MOD., vol. 48, no. 54, 1956, pages 86-91, XP002082741

## Description

L'invention se rapporte à l'utilisation de l'acide cinnamique ou d'au moins l'un de ses dérivés, dans une composition cosmétique en tant qu'agent favorisant la synthèse des lipides de la peau.
En particulier, les compositions de l'invention sont destinées à stimuler la synthèse des lipides totaux de la peau particulièrement ceux de l'épiderme.
L'invention a également pour objet une composition cosmétique comprenant une quantité efficace d'acide cinnamique ou d'au moins l'un de ses dérivés.

La peau humaine est constituée de deux compartiments à savoir un compartiment profond, le derme et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il contient également des vaisseaux sanguins et des fibres nerveuses.

L'épiderme est en contact avec l'environnement extérieur. Son rôle consiste à protéger l'organisme de la déshydratation et des agressions extérieures, qu'elles soient chimiques, mécaniques, physiques ou infectieuses.
L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.
Les cellules constituant l'épiderme sont délimitées par un domaine lipidique intercellulaire. Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides.
Ces lipides sont organisés en structures lamellaires spécifiques dont l'intégrité dépend non seulement de la qualité des fractions présentes mais aussi de leur proportion respective. Cette structure lamellaire des lipides du domaine lipidique intercellulaire de l'épiderme est responsable de la fluidité donc de la souplesse de la peau.
Les lipides sont également responsables des propriétés "barrière" de l'épiderme particulièrement du stratum corneum.
Les lipides épidermiques sont synthétisés principalement dans l'épiderme vivant. Ils sont principalement constitués de phospholipides, de sphingolipides, de cholestérol, d'acides gras libres, de triglycérides, d'esters du cholestérol et d'alcanes.
Les phospholipides sont essentiels pour la constitution des membranes cellulaires. Ils jouent un rôle important dans la médiation des signaux extracellulaires et la formation de chaînes aliphatiques libres utilisées pour la production d'énergie. Ils constituent un réservoir d'acides gras libres nécessaires à la constitution des sphingolipides.
Les sphingolipides (ou céramides) sont essentiels pour le maintien de la structure multilamellaire des lipides intercornéocytaires. Ils sont également essentiels pour les échanges en eau et la fonction "barrière" de l'épiderme.
Le cholestérol joue un rôle primordial dans l'hydratation cutanée et dans la fonction "barrière" de l'épiderme.
Les acides gras libres jouent un rôle majeur dans le maintien de la structure lamellaire des lipides du stratum corneum, mais également dans la constitution des membranes cellulaires où ils sont responsables de la fluidité membranaire mais également de processus physiologiques tels que le fonctionnement de récepteurs ou l'activité enzymatique.

On comprend alors le rôle essentiel que jouent les lipides de la peau et l'importance que revêt leur intégrité.

On sait malheureusement que les lipides de la peau, particulièrement de l'épiderme, sont influencés par des facteurs génétiques, le vieillissement, les régimes alimentaires, les saisons, les facteurs environnementaux, les agressions externes et/ou certaines pathologies (scorbut ou pellagre par exemple). Tous ces facteurs ont pour conséquence d'altérer ou encore de modifier la composition des lipides de la peau ou d'en diminuer la quantité, ce qui conduit invariablement à une peau sèche. On sait par exemple que l'absence de la composante lipidique dans un régime a pour conséquence une peau en mauvaise santé. L'absence de lipides conduit à une détérioration générale de la santé et particulièrement à l'apparition d'une peau écailleuse en augmentation concomitante avec l'augmentation de la perte trans-épidermale en eau.
Les lipides de la peau sont donc essentiels dans le maintien de la "barrière" hydrique de la peau.

On sait aussi que les lipides de l'épiderme ont également une influence sur l'activité de certaines enzymes dans la peau impliquées dans la maturation et dans la desquamation du stratum comeum.
Des variations dans le taux et le type de lipides présents dans le stratum comeum influencent donc la fonction "barrière" du stratum comeum, le contenu en eau et l'état de la peau.

On sait aussi qu'à la ménopause les femmes se plaignent de ce que leur peau tire et qu'elle prend l'aspect d'une "peau sèche", voire de l'apparition d'une xérose. Sans chercher à établir une quelconque théorie, sachant que les lipides de la peau jouent un rôle important dans l'hydratation cutanée et que les déficits hormonaux associés à la ménopause s'accompagnent d'un ralentissement général du métabolisme cellulaire, on peut quand même supposer que la sensation de peau qui tire ou de peau sèche que ressent la femme est notamment liée à une diminution de la quantité des lipides totaux de la peau.

On comprend qu'il est donc important de pouvoir stimuler la synthèse des lipides de la peau pour maintenir et/ou restaurer leur intégrité afin de leur permettre de jouer les rôles importants qui leur sont dévolus.

A cet égard, la demanderesse a de manière surprenante et inattendue découvert que l'acide cinnamique ou ses dérivés présentent la propriété de stimuler la synthèse des lipides, particulièrement des lipides totaux de la peau.

L'acide cinnamique est présent sous forme trans dans les huiles essentielles de basilic ou de cannelle, dans la balsamine du Pérou ou encore dans les feuilles de cacao. La forme cis est présente dans l'huile d'*Alpinia malacensis*.

Dans l'art antérieur, l'acide cinnamique ou ses dérivés sont connus pour être utilisés dans des produits cosmétiques (W.A. Poucher, 'Poucher's perfumes, Cosmetics and Soaps. Volume 1, pp84-89), dans des compositions pour la prévention des escarres (JP 07 242 558), comme actif anti-ultraviolets (US 5 093 109), dans des compositions pour permanente (DE 3 301 515, DE 2 912 477, EP 22 996), dans des lotions capillaires (JP 7 053 401, JP 3 041 413 SU-A-1 311 734), dans des compositions dépigmentantes (JP 5 221 845, JP 1 186 811), comme anti-oxydant (EP 664 290, EP 716 847). Certains acides monohydroxycinnamiques sont utilisés également pour leur effet sur la pousse des cheveux et le vieillissement de la peau, via une stimulation de la différenciation cellulaire (JP-05310526) ; ou pour leur effet blanchissant en tant qu'inhibiteur de l'activité tyrosinase, dans des compositions destinées à un effet rajeunissant (JP-04266807).

A la connaissance de la demanderesse il n'a jamais été décrit dans l'état de la technique l'utilisation de l'acide cinnamique ou de ses dérivés pour stimuler la synthèse des lipides totaux de la peau.

L'invention a donc pour objet l'utilisation dans une composition cosmétique d'une quantité efficace d'acide cinnamique ou d'au moins l'un de ses dérivés, l'acide cinnamique ou la composition étant destiné à stimuler la synthèse des lipides totaux de la peau.
L'acide cinnamique ou ses dérivés peuvent être d'origine naturelle ou synthétique. Par origine naturelle, on entend de l'acide cinnamique, ou ses dérivés, préparés à partir de matériel végétal dans lequel ils se trouvent présents à l'état naturel. Par origine synthétique on entend de l'acide cinnamique, ou ses dérivés, préparés par synthèse chimique ou par biotechnologie.
Ainsi, par la suite dans le texte le terme acide cinnamique s'entend comme désignant de l'acide cinnamique, ou ses dérivés, d'origine naturelle ou synthétique, purifiés ou toute préparation les contenant.

Parmi les dérivés d'acide cinnamique utilisables selon l'invention on peut citer à titre d'exemple les esters, les aldéhydes, les alcools, les acides mono- et poly-hydroxycinnamiques et dérivés.
Préférentiellement, selon l'invention on utilise l'acide cinnamique.
Bien entendu, il est possible d'utiliser selon l'invention l'acide cinnamique ou ses dérivés seuls ou en mélange.

On a vu précédemment que les lipides sont impliqués entre autres dans la fonction barrière de la peau, dans l'hydratation cutanée ou encore dans la souplesse de la peau. On a vu également que la ménopause induit des effets cutanés, plus particulièrement sur les lipides de la peau.

Ainsi, un des aspects de l'invention est donc de proposer l'utilisation dans une composition cosmétique d'une quantité efficace d'acide cinnamique ou d'au moins l'un de ses dérivés, l'acide cinnamique ou la composition étant destiné à renforcer la fonction barrière de la peau. Selon un autre aspect, l'invention a pour objet l'utilisation dans une composition cosmétique d'une quantité efficace d'acide cinnamique ou d'au moins l'un de ses dérivés, l'acide cinnamique ou la composition étant destiné à favoriser l'hydratation cutanée.

Selon encore un autre aspect, l'invention a pour objet l'utilisation dans une composition cosmétique d'une quantité efficace d'acide cinnamique ou d'au moins l'un de ses dérivés, l'acide cinnamique ou la composition étant destiné à renforcer la souplesse de la peau.

Selon encore un autre aspect, l'invention a pour objet l'utilisation dans une composition cosmétique d'une quantité efficace d'acide cinnamique ou de l'un au moins de ses dérivés, l'acide cinnamique ou la composition étant destiné à combattre les effets cutanés de la ménopause.

Selon enfin un autre aspect, l'invention a pour objet l'utilisation dans une composition cosmétique d'une quantité efficace d'acide cinnamique ou de l'un au moins de ses dérivés, l'acide cinnamique ou la composition étant destiné à combattre les effets de la ménopause sur les lipides de la peau.

La quantité d'acide cinnamique, ou de ses dérivés, utilisable selon l'invention, dépend bien évidemment de l'effet recherché et doit être en une quantité efficace pour stimuler la synthèse des lipides de la peau.

A titre d'exemple la quantité d'acide cinnamique utilisable selon l'invention peut aller par exemple de 10⁻⁶% à 10% et de préférence de 10⁻³% à 5% du poids total de la composition.

Il est possible dans l'invention d'utiliser l'acide cinnamique ou l'un de ses dérivés associé à un autre produit stimulant la synthèse des lipides.
Parmi ces autres produits stimulant la synthèse des lipides on peut citer les hormones végétales.

Parmi les hormones végétales on peut citer les auxines telles que l'acide 3-indolacétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile.

Préférentiellement, selon l'invention on utilise l'acide β-naphtoxyacétique.

La peau étant constituée de bien d'autres composants que les lipides, il s'avère intéressant lorsque l'on favorise sa synthèse par de l'acide cinnamique de favoriser en même temps la synthèse de ces autres composants comme par exemple le collagène.

Ainsi, il est aussi possible dans l'invention d'utiliser l'acide cinnamique ou l'un de ces dérivés associé à un autre produit stimulant par exemple la synthèse du collagène.

A cet égard les hormones végétales comme les auxines et particulièrement l'acide β-naphtoacétique peuvent encore être citées.

L'invention a aussi pour objet une composition cosmétique destinée à favoriser la synthèse des lipides totaux de la peau comprenant dans un milieu cosmétiquement acceptable une quantité efficace d'acide cinnamique ou de l'un de ses dérivés.

Le produit stimulant la synthèse des lipides et/ou du collagène peut être utilisé en une quantité comprise entre 10⁻⁶% à 10%, et de préférence entre 10⁻³% à 5% du poids total de la composition.

L'invention a en outre pour objet une composition cosmétique hydratante comprenant dans un milieu cosmétiquement acceptable une quantité efficace d'acide cinnamique ou de l'un de ses dérivés.

L'invention a également pour objet une composition cosmétique assouplissante comprenant dans un milieu cosmétiquement acceptable une quantité efficace d'acide cinnamique ou de l'un de ses dérivés.

L'invention a encore pour objet une composition cosmétique destinée à renforcer la fonction "barrière" de la peau comprenant dans un milieu cosmétiquement acceptable une quantité efficace d'acide cinnamique ou de l'un de ses dérivés.

L'invention a enfin pour objet une composition cosmétique destinée à combattre les effets cutanés de la ménopause, particulièrement les effets de la ménopause sur les lipides de la peau, comprenant dans un milieu cosmétiquement acceptable une quantité efficace d'acide cinnamique ou de l'un de ses dérivés.

Par milieu cosmétiquement acceptable, on entend compatible avec la peau, le cuir chevelu, les muqueuses, les ongles, et les cheveux.

La composition selon l'invention comprend bien évidemment un support cosmétiquement acceptable et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Il est également possible d'utiliser selon l'invention en association avec l'acide cinnamique ou l'un au moins de ses dérivés, des composés choisis parmi
- les hormones végétales ;
- les agents antibactériens tels que les macrolides, les pyranosides et les tétracyclines, et notamment l'érythromycine ;
- les agents antagonistes de calcium, comme le vérapamil et le diltiazem ;
- des capteurs de radicaux OH, tels que le diméthylsulfoxyde ;
- des extraits de végétaux tels que ceux d'lridacés ou de soja, extraits pouvant alors contenir ou non des isoflavones ;
- des extraits de micro-organismes dont en particulier des extraits bactériens comme ceux de bactéries filamenteuses non photosynthétiques ;

A la liste ci-dessus, d'autres composés peuvent également être rajoutés, à savoir par exemple les ouvreurs de canaux potassiques tels que le diazoxyde et le minoxidil, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés.

Selon l'invention, on peut, entre autres, associer de l'acide cinnamique ou au moins l'un de ses dérivés à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase, composés décrits comme étant actifs dans le traitement des peaux sensibles et comme présentant des effets anti-irritants, en particulier vis-à-vis de composés irritants éventuellement présents dans les compositions.

Ainsi, un autre objet de l'invention concerne une composition comprenant une quantité efficace d'acide cinnamique ou d'au moins l'un de ses dérivés et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les antagonistes de substance P, de CGRP ou de bradykinine ou les inhibiteurs de NO synthase.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu), et leurs mélanges.

On peut aussi ajouter des actifs antirides, et notamment des produits tenseurs tels que les protéines végétales et leurs hydrolysats, en particulier l'extrait de protéines de soja vendue sous le nom d'Eleseryl® par la société LSN ou le dérivé d'avoine vendu sous la dénomination Reductine® par la société Silab.

Bien entendu, l'acide cinnamique ou l'un au moins de ses dérivés peut être utilisé dans la préparation de compositions cosmétiques et/ou pharmaceutiques, particulièrement dermatologiques, destinées à stimuler la synthèse des lipides.

D'autres caractéristiques et avantages de l'invention ressortiront mieux des exemples qui suivent donnés à titre illustratif et non limitatif. Dans ce qui suit ou ce qui précède, les proportions sont données en pourcentage pondéral, sauf indications contraires.

### Exemple 1 : Etude de l'effet de l'acide cinnamique sur la synthèse des lipides totaux de la peau.

L'étude est faite par mesure de l'incorporation d'acétate marqué au carbone 14 dans des modèles d'épiderme humain reconstruit vendu par la société Skinethic, dont la composition en lipides totaux est proche de celle de l'épiderme humain normal.
Les cultures d'épiderme humain reconstruit sont effectuées selon recommandation du fournisseur.

L'acide cinnamique, aux concentrations de 10⁻⁵, 10⁻⁶, et 10⁻⁷ M est mis en contact pendant 72 heures avec des épidermes humains reconstruits après 14 jours de culture de ces derniers selon les conditions recommandées par le fournisseur. Le marquage à l'acétate ¹⁴C, ([2-¹⁴C]-acétate de sodium vendu par Amersham, 59 mCi/mmol) est effectué 24 heures après mise en contact du produit à tester avec la culture, soit pendant les 48 dernières heures de culture, à raison de 0,5µCi d'acétate ¹⁴C par culture.

En fin de culture, après lavage des épidermes en tampon phosphate (PBS), les épidermes sont dissociés et les cellules lysées par de l'acide perchlorique à 0,5 M sur de la glace. Les lysats subissent alors une extraction par un mélange méthanol/chloroforme (2:1 ), une centrifugation et de nouveau une extraction sur les culots obtenus dans les mêmes conditions que précédement. Les lipides sont séparés par addition de PBS et de chloroforme (technique de Blight et Byer). La phase organique contenant les lipides est prélevée et la radioactivité incorporée dans cette phase est déterminée par scintillation liquide. La phase organique est ensuite séchée sous flux d'azote. Une chromatographie en couche mince sur plaque sur plaques (Merck K60) en utilisant comme éluant un mélange chloroforme/méthanol/eau (50:18:2,6) pour séparer les phospholipides ou un mélange hexane/éther/acide acétique (15:5,6:0,19) pour séparer les lipides neutres.
Les plaques sont ensuite autoradiographiées pendant 24 heures et les chromatogrammes sont analysé par densitométrie à l'aide du logiciel One-D-Scan de la société Scanalytics.

Les résultats sont évalués par rapport à un témoin constitué par des cellules n'ayant pas été traitées à l'acide cinnamique.

Un témoin positif (trifluopérazine à 10⁻⁴M) connu pour stimuler la synthèse des lipides et un témoin négatif (l'acide rétinoïque à 10⁻⁶M) connu pour inhiber la synthèse des lipides sont introduits dans le test comme référence.

Les résultats de ce test exprimés en pourcentage de stimulation sont présentés dans le tableau suivant.

| Traitement | % |
|---|---|
| Cellules non traitées | 100 |
| Acide cinnamique à 10⁻⁵ M | 113 |
| Acide cinnamique à 10⁻⁶ M | 109 |
| Acide cinnamique à 10⁻⁷ M | 124 |
| Trifluoropérazine à 10⁻⁴ M | 122 |
| Acide rétinoïque à 10⁻⁶ M | 82 |

Ces résultats montrent que l'acide cinnamique stimule de façon significative l'incorporation d'acétate ¹⁴C montrant un effet sur la synthèse des lipides.

### Exemple 2 : Exemples de compositions selon l'invention. Ces compositions sont obtenues par les techniques habituelles couramment utilisées en cosmétique ou en pharmacie.

| Composition 1 : crème de soin | |
|---|---|
| Cire d'abeille | 1,5 % |
| Huile d'amandes d'abricot | 13,0 % |
| Conservateurs | 0,3 % |
| Parfum | 0,4 % |
| Acide β-naphtoxyacétique | 0,01 % |
| Acide cinnamique | 0,01 % |
| Xanthane | 0,5 % |
| Cyclopenta diméthylsiloxane | 5,0 % |
| Eau déminéralisée stérilisée | 69,28 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition 2 : huile corporelle | |
|---|---|
| Huile de vaseline | 47,98 % |
| Huile d'amandes d'abricot | 6,0 % |
| Parfum | 1,0 % |
| Acide β-naphtoxyacétique | 0,01 % |
| Acide cinnamique | 0,01 % |
| Cyclopenta diméthylsiloxane | 45,0 % |

| Composition 3 : lait démaquillant | |
|---|---|
| Palmitate d'éthyl-2 hexyle | 10,5 % |
| Fraction liquide de beurre de karité | 16,5 % |
| Conservateurs | 0,3 % |
| Parfum | 0,15 % |
| Acide β-naphtoxyacétique | 0,01 % |
| Acide cinnamique | 0,01 % |
| Hydroxyde de sodium | 0,04 % |
| Polymère carboxyvinylique | 0,2 % |
| Eau déminéralisée stérilisée | 69,79 % |
| Mélange de cétylstéarylglucoside et d'alcools cétylique et stéarylique | 2,5 % |

| Composition 4 : crème de soin | |
|---|---|
| Cire d'abeille | 1,5 % |
| Huile d'amandes d'abricot | 13,0 % |
| Conservateurs | 0,3 % |
| Parfum | 0,4 % |
| Acide β-naphtoxyacétique | 0,01 % |
| Acide cinnamique | 0,01 % |
| Cinnamate d'éthyle | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

## Revendications

1. Utilisation dans une composition cosmétique d'une quantité efficace d'acide cinnamique ou d'au moins l'un de ses dérivés, l'acide cinnamique ou la composition étant destiné à stimuler la synthèse des lipides de la peau.

2. Utilisation selon la revendication 1, **caractérisée par le fait que** le dérivé d'acides cinnamique est choisi parmi les esters, les aldéhydes, les alcools, les acides mono- et poly-hydroxycinnamiques et dérivés.

3. Utilisation selon' la revendication 1 ou 2, **caractérisée en ce que** l'acide cinnamique, l'un de ses dérivés choisi parmi les aldéhydes et les alcools ou la composition sont destinés à renforcer la fonction barrière de la peau.

4. Utilisation selon la revendication 1 ou 2, **caractérisée en ce que**, l'acide cinnamique, l'un de ses dérivés choisi parmi les aldéhydes et les alcools ou la composition sont destinés à favoriser l'hydratation cutanée.

5. Utilisation selon l'une quelconque des revendications 1, 2 ou 4, **caractérisée en ce que** l'acide cinnamique, l'un de ses dérivés choisi parmi les aldéhydes et les alcools ou la composition sont destinés à renforcer la souplesse de la peau.

6. Utilisation selon la revendication 1 ou 2 **caractérisée en ce que**l"acide cinnamique ou l'un de ses dérivés choisi parmi les aldéhydes et les alcools ou la composition sont destinés à combattre les effets cutanés de la ménopause.

7. Utilisation selon la revendication 1 ou 2, **caractérisé en ce que**, l'acide cinnamique, l'un de ses dérivés choisi parmi les aldéhydes et les alcools ou la composition sont destinés à combattre les effets de la ménopause sur les lipides totaux de la peau.

8. Utilisation selon l'une quelconque des revendications précédentes, **caractérisée par le fait que** la composition est utilisée en application topique sur la peau.

9. Utilisation selon l'une quelconque des revendications précédentes, **caractérisée par le fait que** l'acide cinnamique est présent en une quantité allant de 10⁻⁶% à 10% du poids total de la composition.

10. Utilisation selon l'une quelconque des revendications précédentes, **caractérisée par le fait que** l'acide cinnamique est présent en une quantité allant de 10⁻³% à 5% du poids total de la composition.

11. Utilisation selon l'une quelconque des revendications précédentes, **caractérisée par le fait que** l'acide cinnamique est associé à au moins un autre produit stimulant la synthèse des lipides.

12. Utilisation selon l'une quelconque des revendications précédentes, **caractérisée par le fait que** l'acide cinnamique est associé à au moins un autre produit stimulant la synthèse du collagène.

13. Utilisation selon l'une quelconque des revendications 11 ou 12, **caractérisée par le fait que** l'autre produit stimulant la synthèse des lipides et/ou du collagène est choisi parmi les hormones végétales.

14. Utilisation selon la revendication précédente, **caractérisée par le fait que** l'hormone végétale est une auxine.

15. Utilisation selon. la revendication précédente, **caractérisée par le fait que** l'auxine est choisie parmi l'acide indole-3-acétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile.

16. Utilisation selon la revendication précédente, **caractérisée par le fait que** l'auxine est de l'acide β-naphtoacétique.

17. Utilisation selon l'une quelconque des revendications 11 à 16, **caractérisée par le fait que** l'autre produit stimulant la synthèse des lipides est en une quantité comprise entre 10⁻⁶% à 10%, et de préférence entre 10⁻³% à 5% du poids total de la composition.

18. Composition cosmétique comprenant dans un milieu cosmétiquement acceptable au moins une quantité efficace d'acide cinnamique ou de l'un de ses dérivés choisi parmi les alcools, les aldéhydes ou les acides mono- ou polyhydroxycinnamiques associé à au moins une hormone végétale.

19. Composition selon la revendication 17, **caractérisée en ce que** l'hormone végétale est une auxine.

20. Composition cosmétique comprenant dans un milieu cosmétiquement acceptable au moins une quantité efficace d'acide cinnamique ou de l'un de ses dérivés associé à au moins une auxine.

21. Composition selon la revendication 19 ou 20, **caractérisée en ce que** l'auxine est choisie parmi l'acide indole-3-acétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile.

## Patentansprüche

1. Verwendung einer wirksamen Menge an Zimtsäure oder mindestens eines ihrer Derivate in einer kosmetischen Zusammensetzung, wobei die Zimtsäure oder die Zusammensetzung dafür vorgesehen ist, die Synthesen von Lipiden der Haut zu stimulieren.

2. Verwendung nach Anspruch 1, **dadurch gekennzeichnet, dass** das Zimtsäurederivat unter den Estern, den Aldehyden, den Alkoholen, den Mono- und Polyhydroxyzimtsäuren und deren Derivaten ausgewählt wird.

3. Verwendung nach Anspruch 1 oder 2, **dadurch gekennzeichnet, dass** die Zimtsäure, eines ihrer Derivate, das unter den Aldehyden und den Alkoholen ausgewählt wird, oder die Zusammensetzung dafür vorgesehen ist, die Barrierefunktion der Haut zu verstärken.

4. Verwendung nach Anspruch 1 oder 2, **dadurch gekennzeichnet, dass** die Zimtsäure, eines ihrer Derivate, das unter den Aldehyden und den Alkoholen ausgewählt wird, oder die Zusammensetzung dafür vorgesehen ist, die Hydratisierung der Haut zu verbessern.

5. Verwendung nach Anspruch 1, 2 oder 4, **dadurch gekennzeichnet, dass** die Zimtsäure, eines ihrer Derivate, das unter den Aldehyden und den Alkoholen ausgewählt wird, oder die Zusammensetzung dafür vorgesehen ist, die Geschmeidigkeit der Haut zu verbessern.

6. Verwendung nach Anspruch 1 oder 2, **dadurch gekennzeichnet, dass** die Zimtsäure, eines ihrer Derivate, das unter den Aldehyden und den Alkoholen ausgewählt wird, oder die Zusammensetzung dafür vorgesehen ist, die Auswirkungen der Menopause auf die Haut zu bekämpfen.

7. Verwendung nach Anspruch 1 oder 2, **dadurch gekennzeichnet, dass** die Zimtsäure, eines ihrer Derivate, das unter den Aldehyden und den Alkoholen ausgewählt wird, oder die Zusammensetzung dafür vorgesehen ist, die Auswirkungen der Menopause auf die Gesamtlipide der Haut zu bekämpfen.

8. Verwendung nach einem der vorhergehenden Ansprüche, **dadurch gekennzeichnet, dass** die Zusammensetzung in topischer Anwendung auf der Haut verwendet wird.

9. Verwendung nach einem der vorhergehenden Ansprüche, **dadurch gekennzeichnet, dass** die Zimtsäure in einer Menge enthalten ist, die im Bereich von 10⁻⁶ bis 10 % des Gesamtgewichts der Zusammensetzung liegt.

10. Verwendung nach einem der vorhergehenden Ansprüche, **dadurch gekennzeichnet, dass** die Zimtsäure in einer Menge enthalten ist, die im Bereich von 10⁻³ bis 5 % des Gesamtgewichts der Zusammensetzung liegt.

11. Verwendung nach einem der vorhergehenden Ansprüche, **dadurch gekennzeichnet, dass** die Zimtsäure mit mindestens einer weiteren Substanz kombiniert wird, die die Synthese von Lipiden stimuliert.

12. Verwendung nach einem der vorhergehenden Ansprüche, **dadurch gekennzeichnet, dass** die Zimtsäure mit mindestens einer weiteren Substanz kombiniert wird, die die Kollagensynthese stimuliert.

13. Verwendung nach einem der Ansprüche 11 und 12, **dadurch gekennzeichnet, dass** die weitere Substanz, die die Synthese von Lipiden und/oder die Kollagensynthese stimuliert, unter den pflanzlichen Hormonen ausgewählt wird.

14. Verwendung nach dem vorhergehenden Anspruch, **dadurch gekennzeichnet, dass** es sich bei dem pflanzlichen Hormon um ein Auxin handelt.

15. Verwendung nach dem vorhergehenden Anspruch, **dadurch gekennzeichnet, dass** das Auxin unter Indol-3-essigsäure (IAA), 4-Chlorindol-3-essigsäure (4-Cl-IAA), Phenylessigsäure (PAA), Indol-3-buttersäure (IBA), 2,4-Dichlorphenoxyessigsäure (2,4-D), α-Naphthalinessigsäure (α-NAA), β-Naphthoxyessigsäure, Indol Ethanol, Indol Acetaldehyd und Indol Acetonitril ausgewählt wird.

16. Verwendung nach dem vorhergehenden Anspruch, **dadurch gekennzeichnet, dass** es sich bei dem Auxin um β-Napthoxyessigsäure handelt.

17. Verwendung nach einem der Ansprüche 11 bis 16, **dadurch gekennzeichnet, dass** die weitere Substanz, die die Synthese von Lipiden stimuliert, in einer Menge enthalten ist, die im Bereich von 10⁻⁶ bis 10 % und vorzugsweise 10⁻³ bis 5 % des Gesamtgewichts der Zusammensetzung liegt.

18. Kosmetische Zusammensetzung, die in einem kosmetisch akzeptablen Medium mindestens eine wirksame Menge an Zimtsäure oder eines Zimtsäurederivats, das unter den Alkoholen, den Aldehyden und den Mono- oder Polyzimtsäuren ausgewählt ist, in Kombination mit mindestens einem pflanzlichen Hormon enthält.

19. Zusammensetzung nach Anspruch 17, **dadurch gekennzeichnet, dass** es sich bei dem pflanzlichen Hormon um ein Auxin handelt.

20. Kosmetische Zusammensetzung, die in einem kosmetisch akzeptablen Medium mindestens eine wirksame Menge an Zimtsäure oder eines ihrer Derivate in Kombination mit mindestens einem Auxin enthält.

21. Kosmetische Zusammensetzung nach Anspruch 18 oder 19, **dadurch gekennzeichnet, dass** das Auxin unter Indol-3-essigsäure (IAA), 4-Chlorindol-3-essigsäure (4-Cl-IAA), Phenylessigsäure (PAA), Indol-3-buttersäure (IBA), 2,4-Dichlorphenoxyessigsäure (2,4-D), α-Naphthalinessigsäure (α-NAA), β-Naphthoxyessigsäure, Indol Ethanol, Indol Acetaldehyd und Indol Acetonitril ausgewählt ist.

## Claims

1. Use, in a cosmetic composition, of an effective amount of cinnamic acid or of at least one of its derivatives, the cinnamic acid or the composition being intended to stimulate lipid synthesis in the skin.

2. Use according to Claim 1, **characterized in that** the cinnamic acid derivative is chosen from mono- and polyhydroxycinnamic acids, alcohols, aldehydes, esters and derivatives.

3. Use according to Claim 1 or 2, **characterized in that** cinnamic acid, a derivative thereof chosen from the aldehydes and the alcohols, or the composition, are intended to reinforce the barrier function of the skin.

4. Use according to Claim 1 or 2, **characterized in that** cinnamic acid, a derivative thereof chosen from the aldehydes and the alcohols, or the composition, are intended to promote moisturization of the skin.

5. Use according to any one of Claims 1, 2 and 4, **characterized in that** cinnamic acid, a derivative thereof chosen from the aldehydes and the alcohols, or the composition, are intended to reinforce the suppleness of the skin.

6. Use according to Claim 1 or 2, **characterized in that** cinnamic acid, a derivative thereof chosen from the aldehydes and the alcohols, or the composition, are intended to combat the effects of the menopause on the skin.

7. Use according to Claim 1 or 2, **characterized in that** cinnamic acid, a derivative thereof chosen from the aldehydes and the alcohols, or. the composition, are intended to combat the effects of the menopause on the total lipids of the skin.

8. Use according to any one of the preceding claims, **characterized in that** the composition is used in topical application on the skin.

9. Use according to any one of the preceding claims, **characterized in that** the cinnamic acid is present in an amount ranging from 10⁻⁶% to 10% of the total weight of the composition.

10. Use according to any one of the preceding claims, **characterized in that** the cinnamic acid is present in an amount ranging from 10⁻³% to 5% of the total weight of the composition.

11. Use according to any one of the preceding claims, **characterized in that** the cinnamic acid is combined with at least one other product which stimulates lipid synthesis.

12. Use according to any one of the preceding claims, **characterized in that** the cinnamic acid is combined with at least one other product which stimulates collagen synthesis.

13. Use according to either of Claims 11 and 12, **characterized in that** the other product which stimulates lipid and/or collagen synthesis is chosen from plant hormones.

14. Use according to the preceding claim, **characterized in that** the plant hormone is an auxin.

15. Use according to the preceding claim, **characterized in that** the auxin is chosen from 3-indoleacetic acid (IAA), 4-chloro-3-indoleacetic acid (4-C1-IAA), phenylacetic acid (PAA), 3-indolebutyric acid (IBA), 2,4-dichlorophenoxyacetic acid (2,4-D), α-naphthaleneacetic acid (α-NAA), β-naphthoxyacetic acid, indolethanol, indoleacetaldehyde and indoleacetonitrile.

16. Use according to the preceding claim, **characterized in that** the auxin is β-naphthoxyacetic acid.

17. Use according to any one of Claims 11 to 16, **characterized in that** the other product which stimulates lipid synthesis is in an amount of between 10⁻⁶% and 10%, and preferably between 10⁻³% and 5%, of the total weight of the composition.

18. Cosmetic composition comprising, in a cosmetically acceptable medium, at least an effective amount of cinnamic acid or a derivative thereof chosen from the alcohols, the aldehydes and mono- or polycinnamic acids, combined with at least one plant hormone.

19. Composition according to Claim 17, **characterized in that** the plant hormone is an auxin.

20. Cosmetic composition comprising, in a cosmetically acceptable medium, at least an effective amount of cinnamic acid or a derivative thereof, combined with at least one auxin.

21. Composition according to Claim 18 or 19, **characterized in that** the auxin is chosen from 3-indoleacetic acid (IAA), 4-chloro-3-indoleacetic acid (4-C1-IAA), phenylacetic acid (PAA), 3-indolebutyric acid (IBA), 2,4-dichlorophenoxyacetic acid (2,4-D), α-naphthaleneacetic acid (α-NAA), β-naphthoxyacetic acid, indolethanol, indoleacetaldehyde and indoleacetonitrile.
